# EUROPÄISCHE PATENTANMELDUNG

(11) **EP 4 257 188 A1**
(43) Veröffentlichungstag der Anmeldung: **11.10.2023**
(21) Anmeldenummer: 22167246.2
(22) Anmeldetag: 07.04.2022
(51) Int. Cl.: A61P 17/06, A61K 8/49, A61K 8/60, A61K 8/92, A61K 8/97, A61K 31/70, A61P 17/00, A61Q 19/00, A61Q 19/10, A61K 31/35

(54) **DERMATOLOGISCH WIRKSAME ZUSAMMENSETZUNG MIT CATECHINEN UND FLAVONOIDEN**

(71) Anmelder: BioActive Food GmbH, 23795 Bad Segeberg (DE)
(72) Erfinder: VOLLERT, Henning, 23795 Bad Segeberg (DE)
(74) Vertreter: Uexküll & Stolberg

(57) **Zusammenfassung**

Die Erfindung betrifft eine dermatologische Zusammensetzung, die (a) ein oder mehrere Catechine, (b) ein oder mehrere Kaempferol-Flavonoide, und (c) Phlorizin umfasst. Die Erfindung betrifft die Verwendung der dermatologischen Zusammensetzungen in einem Verfahren zur Vorbeugung oder Behandlung einer Hautkrankheit. In einem weiteren Aspekt betrifft die Erfindung die Verwendung der dermatologischen Zusammensetzungen zur kosmetischen Pflege von empfindlicher oder hyperreaktiver Haut sowie zur Vorbeugung oder kosmetischen Behandlung der Hautalterung.

## Beschreibung

Die Erfindung betrifft eine dermatologische Zusammensetzung, die (a) ein oder mehrere Catechine, (b) ein oder mehrere Kaempferol-Flavonoide, und (c) Phlorizin umfasst. Die Erfindung betrifft die Verwendung der dermatologischen Zusammensetzungen in einem Verfahren zur Vorbeugung oder Behandlung einer Hautkrankheit. In einem weiteren Aspekt betrifft die Erfindung die Verwendung der dermatologischen Zusammensetzungen zur kosmetischen Pflege von empfindlicher oder hyperreaktiver Haut sowie zur Vorbeugung oder kosmetischen Behandlung der Hautalterung.

### GEBIET DER ERFINDUNG

Die vorliegende Erfindung betrifft die Verwendung von neuartigen Kombinationen aus pflanzlichen Polyphenolen und Flavonoiden, die eine besondere Wirkung bei der therapeutischen und kosmetischen Behandlung der Haut zeigen.

Die pathologischen Mechanismen, die das klinische Bild bei empfindlicher oder hyperreaktiver Haut sowie auch bei bestimmten Hauterkrankungen, wie z.B. atopischer Dermatitis, Prurigo, Psoriasis und Ichthyose, prägen, sind in der wissenschaftlichen Literatur umfänglich beschrieben worden. Jedoch sind bei den meisten der genannten Hauterkrankungen die zu Grunde liegenden Ursachen noch weitgehend unbekannt.

Im Falle der atopischen Dermatitis scheinen genetische Prädispositionen ebenso wie Umwelteinflüsse und Lebensgewohnheiten eine signifikante Rolle bei der Entstehung der Erkrankung zu spielen. So wurden u.a. Luftschadstoffe, eine verstärkte Allergen-Exposition, z.B. durch Haustiere oder Hausstaubmilben, sowie auch Ernährungsgewohnheiten und Rauchen als krankheitsfördernde oder sogar krankheitsauslösende Faktoren diskutiert. Nach aktuellen Erkenntnissen könnte beim aktiven atopischen Ekzem ferner auch der grampositive Erreger *Staphylococcus aureus* eine Rolle innerhalb der Pathogenese zu spielen. Es konnte beobachtet werden, dass sich dieser Organismus bei bestimmten Komplikationen der atopischen Dermatitis regelmäßig nachweisen lässt.

In den letzten Jahren verstärkten sich zunehmend auch die Hinweise darauf, dass bestimmte Proteasen die Durchlässigkeit der "Hautbarriere" beim Menschen beeinflussen und regulieren können. Diese Barrierewirkung der Haut ist bei verschiedenen Hauterkrankungen gestört, u.a. bei atopischer Dermatitis und Psoriasis. So wurde insbesondere festgestellt, dass die Häufigkeit des Auftretens von atopischer Dermatitis mit Mutationen im Gen korreliert, welches für die Protease Chymotrpysin kodiert (Vasolipoulos et. al. 2004). Ferner konnte gezeigt werden, dass der Verlust der Barrierewirkung auch mit einer verringerten Expression der Proteasen Fillaggrin, Loricrin, und Involucrin einhergeht (Furue, 2020). Gleichzeitig sind Entzündungsfaktoren, wie beispielsweise IL-1, IL-4 und IL-17 deutlich erhöht (Furue, 2020; Brembilla et al. 2018). Die Verabreichung von spezifischen Antikörpern gegen diese Interleukine kann einen erheblichen Teil der Symptome bei der Psoriasis und der atopischen Dermatitis temporär lindern (Jappe et al. 2021; Yang et al. 2021).

Es besteht ein Bedarf nach Mitteln und Verfahren, die bei empfindlicher oder hyperreaktiver Haut oder bei bestimmten Hauterkrankungen, wie z.B. atopischer Dermatitis, zu einer Verbesserung des Hautbildes und zu einer Verringerung der Symptome wie Entzündung und Juckreiz führen. Die gegenwärtig verfügbaren Möglichkeiten der Behandlung sind nicht zufriedenstellend, da es regelmäßig nur zu temporären Verbesserungen in Bezug auf die Symptome kommt und darüber hinaus eine Reihe von Nebenwirkungen auftreten können.

So erfolgt die Behandlung der atopischen Dermatitis sowie der Psoriasis häufig durch UV-Bestrahlung oder durch Verabreichung von Corticoidsteroide, Calcineurin-Hemmer, Psoralen oder Cyclosporin A. Diese Mittel weisen jedoch bei wiederholter Anwendung signifikante Nebenwirkungen (Werfel, 2003). Auch die Verabreichung von Antikörpern gegen Interleukine wie IL-23 oder IL-4 hat sich als Behandlungsansatz bei Psoriasis etabliert. Diese Antiköper müssen jedoch einerseits injiziert werden und beeinflussen anderseits direkt das Immunsystem des Patienten. Diese systemische Gabe solcher Antikörper kann zu verschiedenen unerwünschten Nebenwirkungen führen, insbesondere zu einem erhöhten Risiko für mikrobielle Infektionen (Daves et al. 2020). Daher wird die Therapie mit Antikörpern meist nur bei schweren Krankheitsbildern eingesetzt werden. Darüber hinaus ist die Therapie mit Antikörpern äußerst kostenintensiv.

Außerdem werden juckreizlindernde Mittel verwendet, insbesondere Antihistaminika mit den Wirkstoffen Clemastin, Promethazin, Hydroxyzin, Dimetinden und Doxylamin. Diese Mittel weisen jedoch häufig unerwünschte Nebenwirkungen auf, wie z.B. Müdigkeit, Verminderung der Reaktionsbereitschaft, paradoxe Reaktionen wie Erregung und Halluzinationen bei Kindern und älteren Personen, Kopfschmerzen und anticholinerge Nebenwirkungen wie Mundtrockenheit, Akkommodationsstörungen, Verstopfung und Harnretention. Es können in seltenen Fällen auch Herz-Kreislauf-Reaktionen wie ein tiefer oder hoher Blutdruck oder Herzrhythmusstörungen auftreten.

Aufgabe der vorliegenden Erfindung ist es, dermatologisch wirksame Zusammensetzungen bereitzustellen, die zur Prophylaxe und Behandlung von empfindlicher oder hyperreaktiver Haut und bestimmten Hauterkrankungen geeignet sind, ohne dabei signifikante Nebenwirkungen aufzuweisen. Diese Zusammensetzungen sollten sich idealerweise für eine dauerhafter Anwendung eignen.

### BESCHREIBUNG DER ERFINDUNG

Es wurde im Rahmen der vorliegenden Erfindung überraschend festgestellt, dass die Kombination aus Catechinen, Kaempferol-Flavonoiden und Phlorizin vorteilhafte synergistische Wirkungen bei der Behandlung von Hautkrankheiten sowie auch bei der kosmetischen Pflege von empfindlicher oder hyperreaktiver Haut zeigt. In einem ersten Aspekt betrifft die Erfindung somit eine dermatologische Zusammensetzung, die folgende Bestandteile umfasst:
(a) ein oder mehrere Catechine,
(b) ein oder mehrere Kaempferol-Flavonoide, und
(c) Phlorizin.

Die dermatologischen Zusammensetzungen der vorliegenden Erfindung umfassen ein oder mehrere Catechine. Catechine sind polyphenolische Pflanzenmetabolite aus der Gruppe der Flavanole. Es handelt sich um hydrierte Flavone oder Anthocyanidine, die in vielen pflanzlichen Geweben als Zwischenstufen bei der Biosynthese von Flavonoiden vorkommen. Catechine kommen in hohen Mengen in vielen Teesorten vor, darunter in Weißtee, Grüntee, Schwarztee und Oolong. Catechine kommen daneben auch in Schokolade, Früchten, Gemüse und Wein vor. Sie bilden die monomeren Bausteine der kondensierten Proanthocyanidine, einer Reihe natürlicher Gerbstoffe, z.B. im schwarzen Tee oder Kakao, wo sie auch zur Geschmacksbildung beitragen.

Es ist erfindungsgemäß bevorzugt, dass die dermatologischen Zusammensetzungen der vorliegenden Erfindung ein Catechin umfassen, das ausgewählt ist aus der Gruppe bestehend aus Epicatechin (EC), Epicatechingallat (ECG), Epigallocatechin (EGC) und Epigallocatechingallat (EGCG). In einer Ausführungsform umfassen die dermatologischen Zusammensetzungen EC. In einer weiteren Ausführungsform umfassen die dermatologischen Zusammensetzungen ECG. In einer weiteren Ausführungsform umfassen die dermatologischen Zusammensetzungen EGC. In einer weiteren Ausführungsform umfassen die dermatologischen Zusammensetzungen EGCG. In einer weiteren Ausführungsform umfassen die dermatologischen Zusammensetzungen EC, ECG, EGC und ECG.

In der wissenschaftlichen Literatur wurden Catechine mit verschiedenen Indikationen wie z.B. Arteriosklerose in Verbindung gebracht. Weiterhin ist bekannt, dass Catechine eine antioxidative Wirkung aufweisen und somit möglicherweise eine präventive Wirkung bei Indikationen wie Diabetes, Krebs und Alzheimer aufweisen könnten. Es wurde ferner beschrieben, dass Catechine aufgrund ihrer antioxidative Wirkung bei Auftragung auf die Haut einen Schutz vor Hautkrebs vermitteln können (Yang et al. 2007). Viele der beschriebenen Wirkungen sind bisher allerdings lediglich auf Basis von in vitro Studien postuliert worden und bedürfen einer finalen Bestätigung in vivo.

Erfindungsgemäß beträgt die Menge an Catechin in der dermatologischen Zusammensetzung vorzugsweise von 0,01% bis 15% (w/w), von 0,1% bis 10% (w/w) oder von 0,5% bis 5% (w/w). Die Menge an Catechin in der dermatologischen Zusammensetzung beträgt vorzugsweise mehr als 0,01%, mehr als 0,1%, mehr als 1%, mehr als 2%, mehr als 3%, mehr als 4%, mehr als 5%, mehr als 6%, mehr als 7%, mehr als 8%, mehr als 9%, mehr als 10%, mehr als 11%, mehr als 12%, mehr als 13%, oder mehr als 14% (w/w), bezogen auf das Gesamtgewicht der erfindungsgemäßen Zusammensetzung.

Die dermatologischen Zusammensetzungen der vorliegenden Erfindung umfassen darüber hinaus ein oder mehrere Kaempferol-Flavonoide. Kaempferol ist ein natürlich vorkommendes Flavonoid, das in roten Weintrauben, Ginkgo, Grapefruits und in verschiedenen Kohlsorten wie Brokkoli, Grünkohl und Rosenkohl vorkommt. Kaempferol hat die folgende Strukturformel (I).

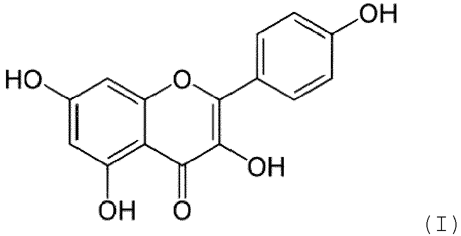

Als Kaempferol-Flavonoide gelten neben Kaempferol auch solche Flavonoide, die von der obigen Struktur abgeleitet sind, z.B. durch Substitution von H-Atomen oder OH-Gruppen. Bei den Kaempferol-Flavonoiden, die in den dermatologischen Zusammensetzungen der vorliegenden Erfindung verwendet werden, kann es sich insbesondere um Glycoside von Kaempferol handeln. Als Glycoside von Kaempferol werden solche Derivate der obigen Formel (I) verstanden, die mindestens eine Zucker-Gruppe umfassen, welche an das Kaempferol gebunden ist. Glycoside im Sinne der vorliegenden Erfindung sind Verbindungen, die einen glycosidisch gebundenen Zuckerrest umfassen und die allgemeine Struktur R-O-Z besitzen, wobei Z der Zuckerrest und R das Aglycon ist. Jede Art von Zucker, der über eine glycosidische Bindung an Formel I binden kann, kann erfindungsgemäß eingesetzt werden. Die glycosidisch gebundene Gruppe kann z.B. ein Mono- oder ein Polysaccharid, wie z.B. ein Disaccharid oder ein Trisaccharid, sein.

Es ist erfindungsgemäß bevorzugt, dass die glycosidisch gebundene Gruppe ein Monosaccharid ist. So kann es sich z.B. bei dem Zucker, welcher glycosidisch an das Kaempferol gemäß Formel I gebunden ist, um ein Monosaccharid handeln, das ausgewählt ist aus der Gruppe bestehend aus Glucose, Fructose, Rhamnose, Glucopyranose, Mannose, und Galactose. Das Monosaccharid ist vorzugsweise Glucose. In einer anderen Ausführungsform handelt es sich bei der Zucker-Gruppe, die glycosidisch an die Verbindung der Formel I gebunden ist, um ein Polysaccharid. Geeignet Polysaccharide umfassen z.B. Di-, Tri-, Tetra- und Pentasaccharide. Geeignete Disaccharide sind z.B. Cellobiose, Gentiobiose, Isomaltose, Isomaltulose, Lactose, Lactulose, Laminaribiose, Maltose, Maltulose, Melibiose, Neohesperidose, Neotrehalose, Nigerose, Rutinose, Sambubiose, Sophorose, Saccharose, und Trehalose. Geeignete Trisaccharide sind z.B. Fucosidolactose, Gentianose, Isokestose (1-Kestose), Kestose (6-Kestose), Maltotriose, Manninotriose, Melezitose, Neokestose, Panose, Raffinose, und Umbelliferose. In einer bevorzugten Ausführungsform ist das an das Flavonol gebundene Polysaccharid Rutinose.

Die glycosidisch gebundene Zucker-Gruppe ist vorzugsweise beta-glycosidisch an das Flavonol gebunden. Dabei ist es besonders bevorzugt, dass die glycosidische Bindung der Zucker-Gruppe über eine der in Formel I gezeigten OH-Gruppen in Position 3', 4', 3, 5 oder 7 erfolgt. Die Nummerierung der Positionen in dem Flavonol folgt der allgemein bekannten chemischen Nomenklatur, wie es in der folgenden Formel (II) angegeben ist:

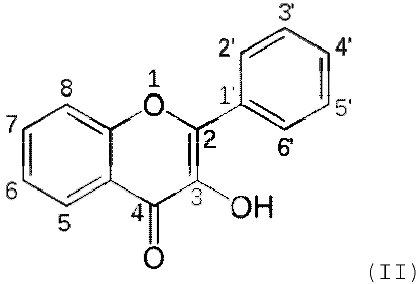

In einer bevorzugten Ausführungsform befindet sich die glycosidisch gebundene Gruppe des Inhibitors in Position 7 von Kaempferol. In einer weiteren bevorzugten Ausführungsform befindet sich die glycosidisch gebundene Gruppe des Inhibitors in Position 3 von Kaempferol. In einer noch weiteren bevorzugten Ausführungsform umfasst der Inhibitor nur eine einzige glycosidisch gebundene Zucker-Gruppe, die sich in Position 3 von Kaempferol befindet. In einer noch weiteren bevorzugten Ausführungsform umfasst der Inhibitor nur eine einzige glycosidisch gebundene Zucker-Gruppe, die sich in Position 7 von Kaempferol befindet. Das bedeutet, dass der Inhibitor in diesen Ausführungsformen zusätzlich zu der glycosidisch gebundenen Gruppe in Position 3 oder 7 von Kaempferol keine weitere glycosidisch gebundene Gruppe enthält.

Erfindungsgemäß kann das Kaempferol-Glycosid auch mehrere Zucker-Gruppen umfassen, die jeweils über eine glycosidische Bindung an das Kaempferol gemäß Formel I gebunden sind. Das Kaempferol-Glycosid kann z.B. 2, 3, 4, 5 oder mehr derartig gebundene Zucker-Gruppen umfassen. Diese glycosidisch gebundenen Zucker sind vorzugsweise über eine der in Formel I gezeigten OH-Gruppen in Position 3', 4', 3, 5 oder 7 gebunden.

Bevorzugte Kaempferol-Glycoside, die in den dermatologischen Zusammensetzungen der vorliegenden Erfindung verwendet werden können umfassen Kaempferol-3-O-Glucosid, Kaempferol-3-O-rutinosid, Kaempferol-3,7-di-O-α-L-rhamnosid, Kaempferol-3-(2G-xylosylrutinosid), 3-Glucosylkaempferol, Dihydrokaempferol-3-rhamnosid, 4'-Methylkaempferol, Kaempferol-3-O-feruloyl-diglucosid-7-O-glucosid, Kaempferol-3-O-hydroxyferuloyl-diglucosid, Kaempferol-3-O-disinapoyl-triglucosid-7-O-diglucosid, Kaempferol-3-O-sinapoyl-triglucosid, Kaempferol-3-O-sinapoyl-diglucosid, Kaempferol-3-O-disinapoyl-triglucosid-7-O-glucosid, Kaempferol-3-O-diglucosid-7-O-diglucosid, Kaempferol-3-O-triglucosid-7-O-glucosid, Kaempferol-3-O-glucosid-7-O-glucosid, Kaempferol-3-O-triglucosid, Kaempferol-3-O-diglucosid, Kaempferol-3-O-glucosid, und ähnliche. In einer besonders bevorzugten Ausführungsform ist das Kaempferol-Glycosid, welches in den dermatologischen Zusammensetzungen der vorliegenden Erfindung verwendet wird, Kaempferol-7-O-Glucosid.

Die glycosidisch an Kaempferol gebundene Zucker-Gruppe kann weitere Modifikationen aufweisen. So ist die Zucker-Gruppe in einer Ausführungsform acyliert, d.h. die Zucker-Gruppe umfasst eine oder mehrere Acylgruppen. Bevorzugte, acylierte Kaempferol-Glycoside sind z.B. ausgewählt aus der Gruppe bestehend aus Kaempferol-3-O-hydroxyferuloyl-tetraglucosid, Kaempferol-3-O-hydroxyferuloyl-diglucosid-7-O-hydroxyferuloyl-diglucosid, Kampferol-3-O-hydroxyferuloyl-diglucosid-7-O-glucosid, Kaempferol-3-O-sinapoyl-diglucosid-7-O-diglucosid und Kaempferol-3-O-sinapoyl-diglucosid-7-O-glucosid. In einer besonders bevorzugten Ausführungsform ist das acylierte Kaempferol-Glycosid Kaempferol-3-O-sinapoyl-diglucosid-7-O-diglucosid. Es ist erfindungsgemäß bevorzugt, dass es sich bei der glycosidisch an Kaempferol gebundene Zucker-Gruppe um ein acyliertes Polysaccharid handelt.

Darüber hinaus kann eine an Kaempferol gebundene Zucker-Gruppe ein Zimtsäurederivat sein, d.h. die Zucker-Gruppe umfasst eine oder mehrere von der Zimtsäure abgeleitete Gruppen, wie z.B. eine Hydroxzimtsäuregruppe, wie z.B. eine Sinapinsäuregruppe (3,5-Dimethoxy-4-hydroxy-zimtsäure), α-Cyano-4-hydroxy-zimtsäuregruppe (HCCA), Ferulasäuregruppe (4-Hydroxy-3-methoxyzimtsäure) oder Kaffeesäuregruppe. In dieser Ausführungsform liegt die Zucker-Gruppe z.B. als Sinapoyl-Glycosid oder als Feruloyl-Glycosid vor.

Erfindungsgemäß beträgt die Menge an Kaempferol-Flavonoiden in der dermatologischen Zusammensetzung vorzugsweise von 0,01% bis 15% (w/w), von 0,1% bis 10% (w/w) oder von 0,5% bis 5% (w/w). Die Menge an Kaempferol oder Kaempferol-Glucosid in der dermatologischen Zusammensetzung beträgt vorzugsweise mehr als 0,01%, mehr als 0,1%, mehr als 1%, mehr als 2%, mehr als 3%, mehr als 4%, mehr als 5%, mehr als 6%, mehr als 7%, mehr als 8%, mehr als 9%, mehr als 10%, mehr als 11%, mehr als 12%, mehr als 13%, oder mehr als 14% (w/w), bezogen auf das Gesamtgewicht der erfindungsgemäßen Zusammensetzung.

Die Mengen der jeweiligen Komponenten der erfindungsgemäßen dermatologischen Zusammensetzung können durch Standardverfahren ermittelt werden, wie z.B. durch Hochleistungsflüssigkeitschromatographie (HPLC) oder Massenspektrometer (MS).

Das Kaempferol-Flavonoid stammt vorzugsweise aus einer Pflanze der Gattung Brassica. Bei der Gattung Brassica handelt es sich um eine Gattung innerhalb der Familie der Kreuzblütengewächse (Brassicaceae). Besonders bevorzugt ist es, dass die Kaempferol-Flavonoide der erfindungsgemäßen Zusammensetzung ausgehend von Pflanzen der Spezies Brassica oleracea gewonnen wird. Bei der Spezies Brassica oleracea handelt es sich um eine formenreiche Pflanzenart innerhalb der Gattung Brassica. Die Art umfasst zahlreiche Sorten, zu denen u.a. Brokkoli (Brassica oleracea var. silvestris L.), Rotkohl (Brassica oleracea var. capitata f. rubra L.), Weißkohl (Brassica oleracea var. capitata f. alba), Wirsing (Brassica oleracea L. convar. capitata (L.) Alef. var. sabauda L.), Chinakohl (Brassica rapa ssp. pekinensis) und Grünkohl (Brassica oleracea convar. acephala var. sabellica) zählen. Besonders bevorzugt ist es, dass das Kaempferol oder Kaempferol-Glucosid der dermatologischen Zusammensetzung aus Grünkohl stammt.

Die dermatologischen Zusammensetzungen der vorliegenden Erfindung umfassen ferner das Flavonoid Phlorizin.

Erfindungsgemäß beträgt die Menge an Phlorizin in der dermatologischen Zusammensetzung vorzugsweise von 0,01% bis 15% (w/w), von 0,1% bis 10% (w/w) oder von 0,5% bis 5% (w/w). Die Menge an Phlorizin in der dermatologischen Zusammensetzung beträgt vorzugsweise mehr als 0,01%, mehr als 0,1%, mehr als 1%, mehr als 2%, mehr als 3%, mehr als 4%, mehr als 5%, mehr als 6%, mehr als 7%, mehr als 8%, mehr als 9%, mehr als 10%, mehr als 11%, mehr als 12%, mehr als 13%, oder mehr als 14% (w/w), bezogen auf das Gesamtgewicht der erfindungsgemäßen Zusammensetzung.

Die dermatologische Zusammensetzung der vorliegenden Erfindung kann neben den oben genannten Catechinen und Flavonoiden weitere Substanzen umfassen, die regelmäßig in Produkten für die dermatologische Anwendung enthalten sind. So kann die dermatologische Zusammensetzung der vorliegenden Erfindung mindestens einen dermatologisch akzeptablen Träger umfassen. Der Träger kann in der Zusammensetzung in einer Menge von 30% bis 99%, vorzugsweise von 40% bis 98% (w/w), von 50% bis 97% (w/w), von 60% bis 96% (w/w) oder von 70% bis 95% (w/w) enthalten sein, bezogen auf das Gesamtgewicht der erfindungsgemäßen Zusammensetzung. Der Träger wird so gewählt werden, dass er keine Allergien oder ähnliche abträglichen Reaktionen der Haut auslöst. Insbesondere wird der dermatologisch verträgliche Träger die Haut nicht reizen oder anderweitig nachteilig beeinflussen. Auch eine negative Auswirkung auf andere Bestandteile der Zusammensetzung sollte ausgeschlossen sein. So beeinträchtigt ein dermatologisch verträglicher Träger die Aktivität, Löslichkeit oder Dispergierbarkeit anderer Inhaltsstoffe in der Zusammensetzung nicht.

Die erfindungsgemäßen dermatologischen Zusammensetzungen können in verschiedenen Formen vorliegen. Sie können als wässrige Lösungen, Emulsionen, Dispersionen oder Feststoffe vorliegen. In einer bevorzugten Ausführungsform ist liegt die erfindungsgemäße dermatologische Zusammensetzung als Emulsion vor. Die Emulsion kann eine Öl-in-Wasser-Emulsion (O/W), eine Wasser-in-Öl-Emulsion (W/O) oder eine Wasser-in-Öl-in-Wasser-Emulsion (W/O/W) sein. Die Emulsion kann einen oder mehrere anionische, kationische oder nichtionische Emulgatoren enthalten, z.B. in einer Menge von 1% bis etwa 5%, bezogen auf das Gewicht der Emulsion.

In einer weiteren bevorzugten Ausführungsform liegt die erfindungsgemäße dermatologische Zusammensetzung als Lösung oder Lotion vor. Sind die dermatologischen Zusammensetzungen als Lösungen bzw. Lotionen formuliert, werden vorzugsweise Öle, wie beispielsweise Sanddornöl, Borretschöl, Mandelöl, Rapsöl und ähnliche pflanzliche oder tierische Öle als Lösungsmittel verwendet. Alternativ können auch Fette, Wachse und andere natürliche oder synthetische Fettkörper als Lösungsmittel verwendet werden, vorzugsweise Ester von Fettsäuren mit Alkoholen niedriger C-Zahl, wie beispielsweise Glycerin, Isopropanol, Propylenglykol oder Ester von Fettalkoholen mit Alkansäuren niedriger C-Zahl oder mit Fettsäuren. Alternativ können auch Alkohole, Diole oder Polyole niedriger C-Zahl, sowie deren Ether, als Lösungsmittel verwendet werden, vorzugsweise Ethanol, Propylenglykol, Isopropanol, Glycerin, Ethylenglykol, Ethylenglykol-Inoethylether, Ethylenglykol-Monobutylether, Propylenglykol-Monomethylether, Propylenglykol-Nonoethylether oder Propylenglykol-Inonobutylether, Diethylenglykol-Monomethylether, Diethylenglykol-Inonoethylether und vergleichbare Produkte. Wasser oder wässrige Lösungen können ebenfalls als Lösungsmittel verwendet werden. Die genannten Lösungsmittel könnten auch als Gemische eingesetzt werden. So kann beispielsweise Wasser den alkoholischen Lösungsmitteln zugesetzt werden.

In einer weiteren bevorzugten Ausführungsform liegt die erfindungsgemäße dermatologische Zusammensetzung als Gel oder Hydrogel vor. Gele entsprechend der Erfindung enthalten üblicherweise Alkohole (z.B. Isopropanol, Ethanol, 1,3-Propandiol, Glycerin) und Wasser bzw. eines der oben genannten Öle, oftmals in Gegenwart eines Verdickungsmittels, welches bei öligalkoholischen Gelen vorzugsweise Aluminiumsilikat oder Siliziumdioxid, bei wässrig-alkoholischen oder alkoholischen Gelen vorzugsweise ein Polyacrylat sein kann. Die dermatologische Zusammensetzung kann auch als fester Stift formuliert sein. Feste Stifte können z.B. synthetische und/oder natürliche Wachse, Fettalkohole und/oder Fettsäureester enthalten.

Die erfindungsgemäßen dermatologischen Zusammensetzungen können auch als Hautreinigungsmittel oder Shampoonierungsmittel formuliert sein. Solche Mittel enthalten vorzugsweise mindestens eine anionische, nicht-ionische oder amphotere oberflächenaktive Substanz oder auch Gemische aus solchen Substanzen. Die oberflächenaktive Substanz bzw. die Gemische aus diesen Substanzen können in einer Konzentration zwischen 0,01% und 50 % (w/w) in dem Hautreinigungsmittel oder Shampoonierungsmittel vorliegen.

Die erfindungsgemäße Zusammensetzung kann ferner zusätzliche Inhaltsstoffe enthalten, die üblicherweise in dermatologischen Produkten verwendet werden. Solche Inhaltsstoffe werden zum Beispiel im International Cosmetic Ingredient Dictionary and Handbook, 16. Edition (2016), Personal Care Products Council, Washington, D.C., ausführlich erörtert. Die erfindungsgemäße Zusammensetzung kann beispielsweise ein oder mehrere Antioxidantien, Bindemittel, pH-Mittel, Puffer, Farbstoffe, Verdickungsmittel, Weichmacher, Anti-Schaummittel, Feuchthaltemittel, Konservierungsmittel, Bakterizide, Emulgatoren, Tenside, Pigmente, Farbpigmente, Fette, Öle, Wachse, ätherische Öle oder Duftstoffe enthalten.

In einer besonders bevorzugten Ausführungsform der vorliegenden Erfindung umfasst die dermatologische Zusammensetzung Arginin oder ein Salz, Ester oder Amid von Arginin, um den Feuchtigkeitsgehalt in der Haut zu erhöhn. In einer weiteren besonders bevorzugten Ausführungsform der vorliegenden Erfindung umfasst die dermatologische Zusammensetzung Harnstoff. Die Verwendung von Harnstoff ist besonders bevorzugt, da Harnstoff einerseits wasserbindende Eigenschaften hat und andererseits die Aufnahme anderer kosmetischer Inhaltsstoffe verbessert. Die erfindungsgemäße Zusammensetzung kann eine Menge von 1% bis 5% (w/w) Harnstoff umfassen, vorzugsweise eine Menge von 2% bis 4% (w/w), wie z.B. 3% (w/w), bezogen auf das Gesamtgewicht der erfindungsgemäßen Zusammensetzung.

In einer weiteren bevorzugten Ausführungsform umfasst die Zusammensetzung der vorliegenden Erfindung eine Verbindung, die zum Einstellen des pH-Werts dient. Da die erfindungsgemäße Zusammensetzung zur Auftragung auf die menschliche Haut vorgesehen ist, sollte sie einen leicht sauren pH-Wert aufweisen. Die Zusammensetzung weist vorzugsweise einen pH-Wert im Bereich von 2,5 bis 6,5, vorzugsweise von 4,0 bis 6,0 von 5,0 bis 6,0 oder von 5,5 bis 6,0 auf. Der saure pH-Wert kann dadurch erreicht werden, dass man der erfindungsgemäßen dermatologischen Zusammensetzung eine Säure zugesetzt wird, z.B. eine Carbonsäure, und insbesondere eine Alphahydroxysäure. Die Art der Säure, die in der erfindungsgemäßen Zusammensetzung verwendet werden kann, ist in keiner Weise eingeschränkt. Geeignete Säuren umfassen Ameisensäure, Essigsäure, Propionsäure, Valeriansäure, Capronsäure, Caprylsäure und dergleichen. In einer besonders bevorzugten Ausführungsform enthält die Zusammensetzung Milchsäure als Mittel zur Einstellung des pH-Werts. Für die Anwendung auf der menschlichen Haut ist Milchsäure besonders nützlich, da diese auch von den Bakterien der Hautflora produziert und sezerniert wird, um das schützende saure Milieu auf der menschlichen Hautoberfläche zu gewährleisten.

Die dermatologische Zusammensetzung der vorliegenden Erfindung kann ferner ein oder mehrere Emollientien enthalten. Emollientien sind Bestandteile in dermatologischen Produkten, die die Haut weich und geschmeidig machen sollen. Geeignete Emollientien zur Verwendung in den Zusammensetzungen der vorliegenden Erfindung umfassen unter anderem Olivenöl, Palmöl, Sojabohnenöl, Sesamöl, Rapsöl, Nachtkerzenöl, Sonnenblumenöl, Avocadoöl, Olivenöl, Kokosnussöl, Rizinusöl und Distelöl. In einigen Ausführungsformen kann die Zusammensetzung zwei oder mehr verschiedene Emollientien enthalten. Die erfindungsgemäße dermatologische Zusammensetzung kann eine Menge von 1% bis 5% (w/w) an Emollientien enthalten, vorzugsweise eine Menge von 2% bis 4% (w/w), wie z.B. 3% (w/w), bezogen auf das Gesamtgewicht der erfindungsgemäßen Zusammensetzung.

Die dermatologische Zusammensetzung der vorliegenden Erfindung kann ferner ein oder mehrere Befeuchtungsmittel enthalten. Diese Mittel sind werden dermatologischen Produkten regelmäßig zugesetzt, um einen ausreichend hohen Feuchtigkeitsgrad in der Haut zu gewährleisten. Geeignete Befeuchtungsmittel zur Verwendung in den Zusammensetzungen der vorliegenden Erfindung umfassen Glycerin, Aminosäuren, wie Prolin oder Arginin, Zucker, wie Glucose, Mannose und Trehalose, oder andere Verbindungen wie Propylenglycol, Diglycerin, Glycerinmonopropoxylat, Natriumhyaluronat, Natriumpolyaspartat, Natriumpolyglutamat, Sorbeth 20, Sorbeth 6 und hydrierte Stärkehydrolysate. Die dermatologische Zusammensetzung der vorliegenden Erfindung kann eine Menge von 1% bis 5% (w/w) an Befeuchtungsmittel enthalten, vorzugsweise eine Menge von 2% bis 4% (w/w), wie z.B. 3% (w/w), bezogen auf das Gesamtgewicht der erfindungsgemäßen Zusammensetzung.

Die dermatologische Zusammensetzung der vorliegenden Erfindung kann ferner ein oder mehrere Substanzen umfassen, welche die Penetration der Haut fördern. Derartige Substanzen umfassen beispielsweise DMSO, Azone, Ölsäure, cis-Alkenfettsäuren, Heptadecansäure, Liposomen, Nanosomen, und Ähnliches.

Die dermatologische Zusammensetzung der vorliegenden Erfindung kann für verschiedene Verabreichungsarten formuliert sein. Es ist besonders bevorzugt, dass die dermatologische Zusammensetzung der vorliegenden Erfindung vorzugsweise für die topische Anwendung formuliert ist, was bedeutet, dass die Zusammensetzung in einer Form bereitgestellt wird, die es dem Verbraucher ermöglicht, die Zusammensetzung nach Entnehmen aus einem Behältnis, wie z.B. einem Spender oder einer Tube, auf die Haut aufzutragen.

Vorzugsweise ist die dermatologische Zusammensetzung der vorliegenden Erfindung als Flüssigkeit, Salbe, Creme, Peeling, Lotion, Paste, Gel, Hydrogel, Schaum oder Pulver formuliert. Wenn die Zusammensetzung als Flüssigkeit formuliert ist, kann sie in einem Pumpspender verpackt werden, der es ermöglicht, die Flüssigkeit auf die zu behandelnden Hautbereiche zu sprühen. Die erfindungsgemäße dermatologische Zusammensetzung kann auch in ein Pflaster eingearbeitet werden, das auf die Haut aufgeklebt wird.

Die zu behandelnde Haut kann sowohl die Haut des Gesichts als auch die Haut des Körpers umfassen, z.B. die Haut von Hals, Brust, Rücken, Armen, Händen, Beinen oder Oberschenkeln. Gemäß einer bevorzugten Ausführungsform ist die mit der Zusammensetzung der vorliegenden Erfindung zu behandelnde Haut die Gesichtshaut. Gemäß einer anderen bevorzugten Ausführungsform ist die mit der erfindungsgemäßen Zusammensetzung zu behandelnde Haut der Unterarme, Oberarme, Unterschenkel und/oder Oberschenkel.

Die dermatologischen Zusammensetzungen der vorliegenden Erfindung können für die einfache oder mehrfache Aufbringung auf die zu behandelnde Hautpartie, z.B. das Gesicht, vorgesehen werden. So können die Zusammensetzungen mindestens einmal täglich, zweimal täglich, dreimal täglich oder häufiger aufgetragen werden. Bei zweimal täglicher Anwendung liegen zwischen der ersten und der zweiten Anwendung vorzugsweise mindestens 6 Stunden, vorzugsweise 8 Stunden. Typischerweise werden die dermatologischen Zusammensetzungen einmal morgens und einmal abends aufgetragen. Die dermatologischen Zusammensetzungen können über lange Zeiträume hinweg angewendet werden, ohne dass es zu unerwünschten Nebenwirkungen kommt. So kann die Behandlungsdauer beispielsweise mindestens 1 Woche, mindestens 2 Wochen, mindestens 3 Wochen, mindestens 4 Wochen, mindestens 6 Wochen, mindestens 12 Wochen, mindestens 24 Wochen oder länger betragen. In einigen Ausführungsformen kann die Behandlung mit den dermatologischen Zusammensetzungen der vorliegenden Erfindung über mehrere Monate erfolgen, z.B. über 4 Monate, 6 Monate, 8 Monate, 12 Monate, 18 Monate oder 24 Monate.

Wie in den nachfolgenden Beispielen beschrieben wird, waren die dermatologischen Zusammensetzungen der vorliegenden Erfindung in der Lage, die Expression der Proteasen Fillaggrin, Loricrin, und Involucrin in Cytokin-stimulierten Dermatozyten zu erhöhen. Diese Marker sind mit einer funktionstüchtigen Barrierefunktion der Haut assoziiert. Durch Stimulation der Dermatozyten mit proinflammatorischen Cytokinen wurde die Expression der Marker zuvor verringert, um einen entzündlichen Hautzustand nachzustellen. Wie den nachfolgenden Beispielen zu entnehmen ist, kommt es bei Anwendung der erfindungsgemäßen Zusammensetzung zu einer synergistischen Verstärkung der Expression dieser Marker.

Gleichzeitig wird die Expression von Entzündungsmarkern bei Anwendung der erfindungsgemäßen Zusammensetzung verringert. So wird die Expression von IL-24 und CCL-26 signifikant reduziert. IL-24 wird von verschiedenen Zelltypen gebildet, wie beispielsweise von Mastzellen, NK-Zellen, Keratinozyten und bronchiale Epithelzellen. IL-24 spielt bei der Pathogenese von pro-inflammatorischen Autoimmunerkrankungen, wie der Psoriasis und der Arthritis, und bei allergischen Reaktionen und bei atopischer Neurodermitis eine wichtige Rolle. CCL-26 wird u.a. in Fibroblasten, Endothelzellen und Keratinozyten gebildet. Es induziert die Infiltration von Th2-Zellen und Eosinophilen und beeinflusst auf diese Weise zahlreiche Entzündungsreaktionen, einschließlich solcher, die bei Neurodermitis und Psoriasis auftreten (Renert-Yuval et al., 2021). Eine Verringerung von IL-24 und CCL-26 verweist auf Besserung entzündlicher Hautzustände.

Es wurde ferner festgestellt, dass die erfindungsgemäße Zusammensetzung zu einer verstärkten Expression von Kollagen Typ I, alpha 1, auch bekannt als Alpha-1-Typ-I-Kollagen, führt. Dieses Protein bildet einen wichtigen Bestandteil der Haut. Bei langfristiger Gabe von Cortison kommt es zu einer verstärkten Bildung von Kollagen Typ I, alpha 1, was insbesondere bei Hauterkrankungen, die mit Cortison behandelt werden vorteilhaft ist.

Die erfindungsgemäße Zusammensetzung verstärkt darüber hinaus auch zu einer verstärkten Expression von OVOL1 (Ovo-like protein). Dieses Protein reguliert die Expression von Filaggrin. Eine Dysfunktion im Protein OVOL1 kann zur Entwicklung u.a. zur Entwicklung einer atopischen Dermatitis führen (Tsuji G. et al., 2018).

Die oben beschriebenen dermatologischen Zusammensetzungen sind insbesondere zur Vorbeugung oder Behandlung einer Hautkrankheit nützlich. Somit betrifft die Erfindung in einem weiteren Aspekt die oben beschriebenen dermatologischen Zusammensetzungen zur Verwendung in einem Verfahren zur Vorbeugung oder Behandlung einer Hautkrankheit. Die Hautkrankheit ist vorzugsweise ausgewählt aus der Gruppe bestehend aus atopischer Dermatits (auch als Neurodermitis oder atopisches Ekzem bekannt), dyshidrotischem Ekzem, seborrhoischer Dermatitis, Photodermatitis, Prurigo, Pruritus, Psoriasis und Ichthyose. Besonders bevorzugt ist die Verwendung der dermatologischen Zusammensetzungen in einem Verfahren zur Vorbeugung oder Behandlung der Neurodermitis.

Die oben beschriebenen dermatologischen Zusammensetzungen sind darüber hinaus zur nicht-medizinischen, kosmetischen Pflege von empfindlicher oder hyperreaktiver Haut nützlich. Somit betrifft die Erfindung in einem weiteren Aspekt die Verwendung der oben beschriebenen dermatologischen Zusammensetzungen zur kosmetischen Pflege von empfindlicher oder hyperreaktiver Haut.

Die oben beschriebenen dermatologischen Zusammensetzungen sind auch zur Bekämpfung Hautalterung verwendbar, da entzündliche Reaktionen auch bei der Bildung beitragen. Somit betrifft die Erfindung in einem weiteren Aspekt die Verwendung der oben beschriebenen dermatologischen Zusammensetzungen zur kosmetischen Behandlung der Hautalterung.

Der Wirkmechanismus der erfindungsgemäßen dermatologischen Zusammensetzungen, ist noch nicht geklärt. Es wird angenommen, dass bei den genannten pathologischen Hautzuständen verschiedene entzündliche Prozesse zu stark ausgeprägt sind und durch die erfindungsgemäßen dermatologischen Zusammensetzungen gelindert und auf das Niveau gesunder Haut reguliert werden. Die dermatologischen Zusammensetzungen können nach ihrer topischen Applikation durch Penetration der haut über die Talg- und Schweißdrüsen, aber auch entlang von Haarschäften oder durch das Stratum corneum in die tieferen Hautschichten gelangen.

Wie es in den Beispielen beschrieben ist, übten die einzelnen Komponenten der erfindungsgemäßen dermatologischen Zusammensetzungen teilweise nur eine schwache Wirkung bei der Behandlung von pathologischen Hautzuständen aus, während bei Kombination der einzelnen Komponenten zu synergistischen Wirkungen führte.

Alle Mengenangaben, Anteile und Prozentanteile sind, soweit es nicht anders angegeben ist, auf das Gewicht bzw. auf das Gesamtgewicht der Zusammensetzung bezogen.

### KURZE BESCHREIBUNG DER FIGUREN

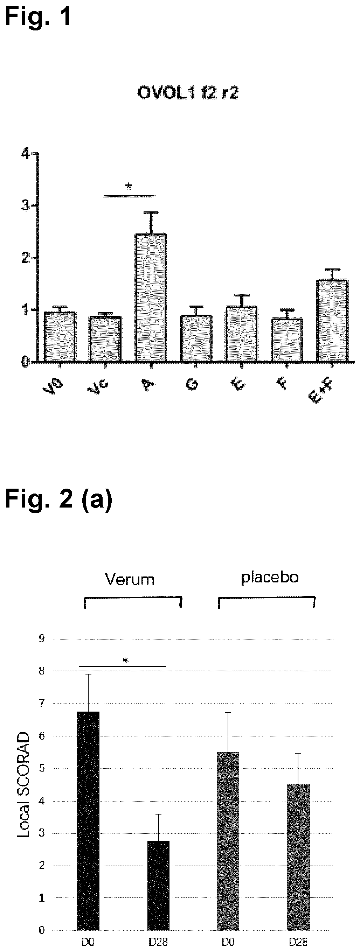

Figur 1 zeigt die Ergebnisse der Messung der relativen Genexpression des Transkriptionsfaktors Ovol1. Vc: stimuliert mit Cytokinen. Vo: nicht stimuliert. Die Keratinozyten wurden darüber hinaus mit Flavonoid-Extrakten inkubiert: G = Grünkohl-Flavonoide, E = Grüntee-Flavonoide, F = Apfel-Flavonoide. A = gleichzeitige Gabe von G, E und F.

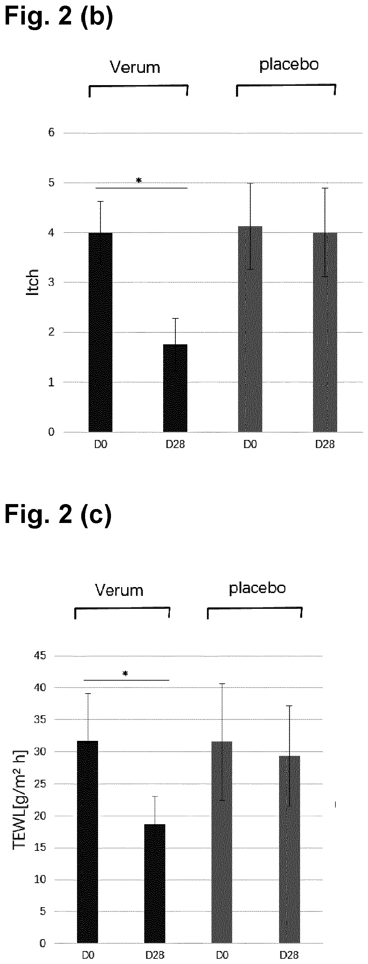

Figur 2 zeigt die Ergebnisse der Messung der SORAD-Werte bei Neurodermitis-Patienten. Es konnte gezeigt werden, dass die Anwendung der erfindungsmäßigen Creme im Vergleich zu einer Placebo-Creme zu einer signifikanten Verbesserung des SORAD-Wertes, zu einer signifikanten Linderung des Juckreizes sowie wirksamen Vermeidung des transepidermalen Wasserverlusts führte.

### BEISPIELE

Die nachfolgenden Beispiele sollen die vorliegende Erfindung erläutern, ohne sie jedoch zu beschränken. Die Beispiele zeigen die dermatologischen Wirkungen der erfindungsgemäßen Zusammensetzungen anhand von in vitro Untersuchungen in Keratinozyten sowie in vivo in einer Patientenstudie.

### Beispiel 1: Genexpressionsuntersuchungen in Keratinozyten

Keratinozyten wurden wie bei Bernard et al. (2012) beschrieben stimuliert, um eine atopische Dermatitis zu simulieren. Zu diesem Zweck wurden differenzierte Keratinozyten mit einer Mischung der Cytokine IL-22 (10 ng/ml), TNF-alpha (10 ng/ml), IL-13 (10 ng/ml) und IL-4 (10 ng/ml) inkubiert. Die Cytokin-stimulierten Zellen wurden anschließend mit einer Verdünnung von jeweils 1:800 der Stocklösungen (50% Ethanol) verschiedener Extrakte inkubiert: 10 mg/ml eines an Kaempferol-Flavonoiden reichen Grünkohl-Extrakts (Anklam Extrakt GmbH, Anklam, Deutschland), 10 mg/ml eines an Catechinen reichen Grüntee-Extrakt (Eurochem Feinchemie GmbH, Deutschland) sowie 10 mg/ml eines an Phlorizin reichen Apfel-Extrakts (Herbstreith & Fox GmbH, Neuenbürg, Deutschland). Als Kontrolle wurden 50% Ethanol verwendet. Die Inkubation erfolgte für 20 h bei 37°C in einer Atmosphäre aus 5% CO₂.

Anschließend wurde der Level der Expression von ausgewählten Genen mittels Real-time PCR bestimmt. Die Gesamt-RNA wurde mittels des Crystal RNAmagic Kits (Bio-Budget Technologies GmbH, Krefeld, Deutschland) nach den Anweisungen des Herstellers isoliert und anschließend in cDNA transkribiert. Anschließend wurde die cDNA mit dem QuantStudio3 System (BD Biosciences Heidelberg, Deutschland) gemäß der Methode von Roth et al. 2014 quantifiziert.

Die PCR wurde analog der Methode von Roth et al. 2014 durchgeführt (Roth et al. 2014). Die folgenden Primer wurden dabei verwendet. Das für den CC Cemokin-Liganden 26 (CCL-26) kodierende Gen wurde mit dem Forward-Primer SEQ ID NO:1 und dem Reverse-Primer SEQ ID NO:2 amplifiziert. Das für das Kollagen Typ 1 alpha 1 Protein (COL1A1) kodierende Gen wurde mit dem Forward-Primer SEQ ID NO:3 und dem Reverse-Primer SEQ ID NO:4 amplifiziert. Das für Filaggrin (FLG) kodierende Gen wurde mit dem Forward-Primer SEQ ID NO:5 und dem Reverse-Primer SEQ ID NO:6 amplifiziert. Das für Interleukin 24 (IL-24) kodierende Gen wurde mit dem Forward-Primer SEQ ID NO:7 und dem Reverse-Primer SEQ ID NO:8 amplifiziert. Das für Involucrin-2 (INV) kodierende Gen wurde mit dem Forward-Primer SEQ ID NO:9 und dem Reverse-Primer SEQ ID NO:10 amplifiziert. Das für Loricrin (LOR) kodierende Gen wurde mit dem Forward-Primer SEQ ID NO:11 und dem Reverse-Primer SEQ ID NO:12 amplifiziert. Das für die carbonische Anhydrase II (CA-2) kodierende Gen wurde mit dem Forward-Primer SEQ ID NO:13 und dem Reverse-Primer SEQ ID NO:14 amplifiziert. Das für das ribosomale Protein L38 (RP38) kodierende Gen wurde mit dem Forward-Primer SEQ ID NO:15 und dem Reverse-Primer SEQ ID NO:16 amplifiziert. Das für das Protein OVOL1 kodierende Gen wurde mit dem Forward-Primer SEQ ID NO:17 und dem Reverse-Primer SEQ ID NO:18 amplifiziert.

Die Ergebnisse der Messungen sind in Tabelle 1 dargestellt. Es lasst sich erkennen, dass die drei Extrakte (G = Grünkohlflavonoide, E = Grünteeflavonoide, F = Apfelflavonoide) allein keine Erhöhung des Markers OVOL1 bewirken. Die Kombination der Extrakte E und F bewirkt eine Erhöhung, aber erst die gleichzeitige Gabe aller 3 Extrakte zeigte eine signifikante Erhöhung der Expression.

**Tab. 1: Ergebnisse der Expressionsexperimente**

| | **Vo** | **Vc** | **G, E, F** | **G** | **E** | **F** | **Marker für** | **Synerg.** |
|---|---|---|---|---|---|---|---|---|
| Loricrin | 1,0 | 0,25 | 1,3 | 0,20 | 0,70 | 0,20 | Hautbarriere | + |
| Flaggrin | 1,0 | 0,18 | 1,2 | 0,16 | 0,60 | 0,19 | Hautbarriere | + |
| Involucrin-2 | 1,0 | 0,50 | 1,50 | 0,40 | 1,0 | 0,60 | Hautbarriere | + |
| | | | | | | | | |
| IL-24 | 1,0 | 4,0 | 0,20 | 4,0 | 0,60 | 4,0 | Entzündung | + |
| CCL-26 | 1,0 | 70 | 1,0 | 38 | 2,0 | 16 | Entzündung | - |
| | | | | | | | | |
| OVOL1 | 1,0 | 0,90 | 2,50 | 0,95 | 1,20 | 0,90 | AHR-Signalkas. | + |
| | | | | | | | | |
| COL1A1 | 1,0 | 0,50 | 2,30 | 0,45 | 1,30 | 0,65 | extr. Matrix | + |

Die Expression von Loricrin, Flaggrin und Involucrin 2 wurde durch wurde durch die drei Extrakte verstärkt. Nach Stimulierung der Keratinozyten mit Cytokinen wird die Expression der Zellen (Vc) im Vergleich zu nicht stimulierten Zellen (Vo) stark herabgesetzt. Die einzelnen Extrakte alleine zeigen keine oder nur eine schwache Normalisierung der Expression. Die gleichzeitige Zugabe der drei Extrakte dagegen ist mit einer synergistischen Wirkung assoziiert und erhöht die Expression der entsprechenden Gene sogar über das Niveau der nicht stimulierten Kontrolle (Vo) hinaus.

Die Verwendung einer Mischung der drei genannten Extrakte zeigte auch eine synergistische Wirkung bei der Verringerung der Expression von IL-24 sowie einen additiven Effekt bei der Verringerung der Expression von CCL-26.

Die Expression von Kollagen und OVOL1 wurde ebenfalls synergistisch verstärkt.

### Beispiel 2: Genexpressionsuntersuchungen in Keratinozyten

Der Nachweis der Wirksamkeit der erfindungsgemäßen Zusammensetzungen wurde in Studien am Menschen erbracht. Dabei wurden die erfindungsgemäßen Zusammensetzungen als Creme formuliert und mit einer Placebo-Creme verglichen.

Als Placebo wurde eine Basiscreme nach Deutschem Arzneimittel-Codex (DAC) verwendet, welche von einem kommerziellen Anbieter (Caesar & Loretz GmbH, Hilden, Deutschland) bezogen wurde. Die Placebo-Creme wies die folgende Rezeptur auf:
- 4,0 g Glycerolmonostearat 60
- 6,0 g of Cetylalkohol
- 7,5 g mittelkettige Triglyceride
- 25,5 g Vaseline(white petroleum jelly)
- 7,0 g Macrogol-20-glycerolmonostearat
- 10,0 g Propylenglykol
- 40,0 g gereinigtes Wasser

Die erfindungsgemäße Creme bestand zu 98,5% (w/w) aus der DAC-Basiscreme und zu jeweils 0,5% (w/w) aus den im Beispiel verwendeten Extrakten von Grüntee, Apfel und Grünkohl.

Die quantitative Bewertung der Wirkung wurde mit Hilfe des SCORAD-Scores ermittelt und dokumentiert. Dabei handelt es sich um ein hinreichend bekanntes klinisches Bewertungssystem, welches regelmäßig genutzt wird um den Schweregrad eines atopischen Ekzems bei einem Patienten möglichst objektiv zu bestimmen. Beobachtet wurden 10 Patienten mit einer moderaten Neurodermitis (SCORAD-Wert < 50). Während der vierwöchigen Studie wurde täglich jeweils morgens und abends der eine Unterarm mit der Placebo-Creme eingecremt, während der andere Unterarm mit der Wirkstoffcreme eingecremt wurde.

Die Ergebnisse der Messung sind in Figur 2 gezeigt. Es lässt sich erkennen, dass sich der lokale SCORAD-Wert im Falle der Wirkstoffcreme über den vierwöchigen Studienzeitraum hinweg signifikant verbesserte. Des Weiteren wurden Juckreiz und transepidermaler Wasserverlust (TEWL) signifikant verringert. Eine solche Verbesserung wurde bei der Placebo-Creme nicht beobachtet.

### LITERATUR

Bernard FX, Morel F, Camus M, Pedretti N, Barrault C, Garnier J, Lecron JC: Keratinocytes under Fire of Proinflammatory Cytokines: Bona Fide Innate Immune Cells Involved in the Physiopathology of Chronic Atopic Dermatitis and Psoriasis. Journal of allergy 2012, 2012:718725.
Brembilla NC, Senra L, Boehncke WH. The IL-17 Family of Cytokines in Psoriasis: IL-17A and Beyond. Front Immunol. 2018 Aug 2;9:1682
Davis J., Ferreira D., Paige E., Gedye C, Boyle M, Infectious Complications of Biological and Small Molecule Targeted Immunomodulatory Therapies. Clin Microbiol Rev. 2020 Jul; 33(3) :
Furue M. Regulation of Filaggrin, Loricrin, and Involucrin by IL-4, IL-13, IL-17A, IL-22, AHR, and NRF2: Pathogenic Implications in Atopic Dermatitis. Int J Mol Sci. 2020, 29;21(15):5382
Furue M, Hashimoto-Hachiya A, Tsuji G. Aryl Hydrocarbon Receptor in Atopic Dermatitis and Psoriasis. Int J Mol Sci. 2019; 20(21):5424
Hrenn A, Steinbrecher T, Labahn A, Schwager J, Schempp CM, Merfort I. Plant phenolics inhibit neutrophil elastase. Planta Med. 2006; 72(12):1127-31.
Jappe U, Beckert H, Bergmann KC, Gülsen A, Klimek L, Philipp S, Pickert J, Rauber-Ellinghaus MM, Renz H, Taube C, Treudler R, Wagenmann M, Werfel T, Worm M, Zuberbier T. Biologics for atopic diseases: Indication, side effect management, and new developments. Allergol Select. 2021 Jan 5;5:1-25.
Kunz B, Oranje AP, Labreze L, Stalder JF, Ring J, Taieb A. Clinical validation and guidelines for the SCORAD index: consensus report of the European Task Force on Atopic Dermatitis. Dermatology. 1997;195(1):10-9.
Kottra G, Daniel H. Flavonoid glycosides are not transported by the human Na+/glucose transporter when expressed in Xenopus laevis oocytes, but effectively inhibit electrogenic glucose uptake. J Pharmacol Exp Ther. 2007, 322(2):829-35
Leikert JF, Räthel TR, Wohlfart P, Cheynier V, Vollmar AM, Dirsch VM. Red wine polyphenols enhance endothelial nitric oxide synthase expression and subsequent nitric oxide release from endothelial cells. Circulation. 2002 Sep 24;106(13):1614-7.
Renert-Yuval Y, Thyssen JP, Bissonnette R, Bieber T, Kabashima K, Hijnen D, Guttman-Yassky E. Biomarkers in atopic dermatitis-a review on behalf of the International Eczema Council. J Allergy Clin Immunol. 2021 Apr;147(4):1174-1190
Roth SA, Simanski M, Rademacher F, Schröder L, Harder J: The pattern recognition receptor NOD2 mediates Staphylococcus aureus-induced IL-17C expression in keratinocytes. The Journal of investigative dermatology 2014, 134(2):374-380.
Vasilopoulos Y, Cork MJ, Murphy R, Williams HC, Robinson DA, Duff GW, Ward SJ, Tazi-Ahnini R. Genetic association between an AACC insertion in the 3'UTR of the stratum corneum chymotryptic enzyme gene and atopic dermatitis. J Invest Dermatol. 2004 Jul;123(1):62-6
Weinreich F, Wood PG, Riordan JR, Nagel G. Direct action of genistein on CFTR, Pflugers Arch. 1997; 434(4): 484-91.
Werfel et al., 2003) Werfel T, Claes C, Kulp W, GreinerW , Graf von der Schulenburg JM, Therapie der Neurodermitis. HTA-Bericht 2003. 46.
Schulze C, Bangert A, Kottra G, Geillinger KE, Schwanck B, Vollert H, Daniel H. Inhibition of the intestinal sodiumcoupled glucose transporter 1 (SGLT1) by extracts and polyphenols from apple reduces postprandial blood glucose levels in mice and humans. Mol Nutr Food Res. 2014 Sep;58(9):1795-808
Severity scoring of atopic dermatitis: the SCORAD index. Consensus Report of the European Task Force on Atopic Dermatitis. Dermatology (Basel, Switzerland) 1993, 186(1):23-31.
Sun P, Vu R, Dragan M, Haensel D, Gutierrez G, Nguyen Q, Greenberg E, Chen Z, Wu J, Atwood S, Pearlman E, Shi Y, Han W, Kessenbrock K, Dai X. OVOL1 Regulates Psoriasis-Like Skin Inflammation and Epidermal Hyperplasia. J Invest Dermatol. 2021; 141(6):1542-1552.
Tsuji G., Ito t., Chiba T., Mitoma C., Nakahara T, Uchi H., Furue M. The role of the OVOL1-OVOL2 axis in normal and diseased human skin. J Dermatol Sci. 2018 Jun;90(3):227-231
Yang CS, Lambert JD, Ju J, Lu G, Sang S. Tea and cancer prevention: molecular mechanisms and human relevance. Toxicol Appl Pharmacol. 2007; 224(3): 265-273.
Yang K, Oak ASW, Elewski BE. Use of IL-23 Inhibitors for the Treatment of Plaque Psoriasis and Psoriatic Arthritis: A Comprehensive Review. Am J Clin Dermatol. 2021 Mar; 22(2):173-192.

## Patentansprüche

1. Dermatologische Zusammensetzung, die folgende Bestandteile umfasst:
(a) ein oder mehrere Catechine,
(b) ein oder mehrere Kaempferol-Flavonoide, und
(c) Phlorizin.

2. Dermatologische Zusammensetzung nach Anspruch 1, wobei die ein oder mehreren Catechine in einer Menge von 0,01% bis 15% (w/w) enthalten sind, bezogen auf das Gesamtgewicht der erfindungsgemäßen Zusammensetzung.

3. Dermatologische Zusammensetzung nach Anspruch 1-2, wobei die ein oder mehreren Kaempferol-Flavonoide in einer Menge von 0,01% bis 15% (w/w) enthalten sind, bezogen auf das Gesamtgewicht der erfindungsgemäßen Zusammensetzung.

4. Dermatologische Zusammensetzung nach Anspruch 1-3, wobei Phlorizin in einer Menge von 0,01% bis 15% (w/w) enthalten sind, bezogen auf das Gesamtgewicht der erfindungsgemäßen Zusammensetzung.

5. Dermatologische Zusammensetzung nach Anspruch 4, wobei das Kaempferol-Flavonoid ausgewählt ist aus der Gruppe bestehend aus Quercetin-7-O-Glucosid und Kaempferol-7-O-Glucosid.

6. Dermatologische Zusammensetzung nach einem der Ansprüche 1-5, wobei die Zusammensetzung von 0,01 bis 10 g Phlorizin umfasst.

7. Dermatologische Zusammensetzung nach einem der Ansprüche 1-6, wobei die Zusammensetzung von 0,01 bis 10 g Kaempferol-Flavonoide umfasst.

8. Dermatologische Zusammensetzung nach einem der Ansprüche 1-7, die ferner Arginin oder ein Salz, Ester oder Amid von Arginin umfasst.

9. Dermatologische Zusammensetzung nach einem der Ansprüche 1-8, die ferner Harnstoff umfasst.

10. Dermatologische Zusammensetzung nach einem der Ansprüche 1-9, die als Flüssigkeit, Salbe, Creme, Peeling, Lotion, Paste, Gel, Hydrogel, Schaum oder Pulver formuliert ist.

11. Dermatologische Zusammensetzung gemäß einem der Ansprüche 1-10 zur Verwendung in einem Verfahren zur Vorbeugung oder Behandlung einer Hautkrankheit.

12. Dermatologische Zusammensetzung zur Verwendung in einem Verfahren gemäß Anspruch 11, wobei die Hautkrankheit ausgewählt ist aus der Gruppe bestehend aus atopischer Dermatits, dyshidrotischem Ekzem, seborrhoischer Dermatitis, Photodermatitis, Prurigo, Pruritus, Psoriasis und Ichthyose.

13. Verwendung einer dermatologischen Zusammensetzung gemäß einem der Ansprüche 1-10 zur kosmetischen Pflege von empfindlicher oder hyperreaktiver Haut.

14. Verwendung einer dermatologischen Zusammensetzung gemäß einem der Ansprüche 1-10 zur kosmetischen Behandlung der Hautalterung.
